# EUROPÄISCHE PATENTSCHRIFT

(11) **EP 3 210 521 B1**
(45) Veröffentlichungstag und Bekanntmachung des Hinweises auf die Patenterteilung: **04.12.2019**
(21) Anmeldenummer: 17156366.1
(22) Anmeldetag: 15.02.2017
(51) Int. Cl.: A61B 1/00, A61B 1/06, G02B 23/24

(54) **ENDOSKOPISCHES SCHAFTINSTRUMENT**
ENDOSCOPIC SHAFT INSTRUMENT
INSTRUMENT À TIGE ENDOSCOPIQUE

(30) Priorität: 24.02.2016 DE 102016202819
(43) Veröffentlichungstag der Anmeldung: 30.08.2017
(73) Patentinhaber: Richard Wolf GmbH, 75438 Knittlingen (DE)
(72) Erfinder: HEIMBERGER, Rudolf, 75038 Oberderdingen (DE)
(74) Vertreter: Patentanwälte Vollmann Hemmer Lindfeld Partnerschaft mbB

(56) Entgegenhaltungen:
- EP-A2- 1 467 416
- CN-U- 204 863 074
- CN-Y- 201 044 740
- DD-A5- 10 387
- DE-A1-102013 201 808
- US-A- 4 778 247
- US-A- 5 337 734
- US-A1- 2007 249 907
- US-A1- 2015 005 580

## Beschreibung

Die Erfindung betrifft ein endoskopisches Schaftinstrument mit den im Oberbegriff von Anspruch 1 angegebenen Merkmalen.

Ausgangspunkt der Erfindung sind solche endoskopischen Instrumente, bei denen im Bereich des distalen Endes eines in den Körper eines Patienten einzuführenden Schaftes elektronische Bauteile angeordnet sind. Diese elektronischen Bauteile können beispielsweise Teil einer Beleuchtungs- oder Beobachtungseinrichtung sowie Teil einer Therapieeinrichtung sein. Die US 2007/0249907 A1 beschreibt ein Endoskop mit distalseitigen LEDs, die unter einer transparenten Plastikkappe angeordnet sind.

In Verbindung mit Instrumenten dieser Art ist es generell erforderlich, einen unbeabsichtigten Strompfad von den elektronischen Bauteilen zu einem mit dem Instrument zu behandelnden Patienten zu verhindern. Bei Instrumenten mit einem metallischen Schaft bzw. mit einem die elektronischen Bauteile radial umgebenden metallischen distalen Endabschnitt des Schaftes kann beispielsweise ein solcher Strompfad von einem elektronischen Bauteil zu dem Patienten über die metallische und damit elektrisch leitende Außenwandung des Schaftes erfolgen. Es ist daher notwendig, im Bereich des distalen Instrumentenendes angeordnete elektronische Bauteile gegenüber der Außenwandung des Schaftes elektrisch zu isolieren. Zu diesem Zweck werden üblicherweise Kunststoffmassen bzw. Kunststoffbauteile verwendet, welche die elektronischen Bauteile außenseitig ummanteln. In Abhängigkeit von einem erforderlichen Isolationswiderstand sowie herstellungsbedingt sind diese Kunststoffummantelungen allerdings vergleichsweise dickwandig, was sich unmittelbar auf die Abmessungen des Schaftes auswirkt. D. h., dass die elektrisch isolierende Ummantelung der elektronischen Bauteile im Normalfall immer einen vergleichsweise großen Schaftquerschnitt bedingt, obwohl es bei endoskopischen Instrumenten eigentlich anzustreben ist, den Schaftquerschnitt möglichst klein zu halten.

Vor diesem Hintergrund liegt der Erfindung die Aufgabe zugrunde, ein endoskopisches Instrument der eingangs beschriebenen Art zu schaffen, welches bei möglichst kleinen lateralen Abmessungen an seinem distalen Endabschnitt eine ausreichende elektrische Isolierung von im Bereich des distalen Endes des Instruments angeordneten elektronischen Bauteilen gewährleistet.

Diese Aufgabe wird durch ein endoskopisches Schaftinstrument mit den in Anspruch 1 angegebenen Merkmalen gelöst, wobei sich vorteilhafte Weiterbildungen dieses Schaftinstrumentes aus den Unteransprüchen, der nachfolgenden Beschreibung sowie aus den Zeichnungen ergeben. Hierbei können die in den Unteransprüchen angegebenen Merkmale vorteilhaft in der angegebenen Kombination, aber auch, soweit technisch sinnvoll, für sich oder in anderer Kombination zur Ausgestaltung der Erfindung beitragen.

Das erfindungsgemäße endoskopische Schaftinstrument weist im Bereich seines distalen Endes, d. h. im Bereich des distalen Endes seines Schaftes zumindest ein elektronisches Bauteil mit einem außenseitig liegenden elektrischen Leiter auf. Das elektronische Bauteil kann z. B. Bestandteil einer in dem distalen Endbereich des Schaftes angeordneten Beleuchtungs-, Beobachtungs- oder Therapieeinrichtung sein. Der außenseitig an dem Bauteil liegende Leiter dient vorzugsweise zur Spannungsversorgung des Bauteils und ist zu diesem Zweck mit einer zu einer Spannungsquelle führenden, durch den Schaft geführten elektrischen Leitung verbunden.

Die Besonderheit der Erfindung besteht darin, dass das zumindest eine elektronische Bauteil zumindest außenumfänglich von einer Glashülse umgeben ist. Weist das erfindungsgemäße Schaftinstrument im Bereich des distalen Endes seines Schaftes mehrere gegenüber der Außenumgebung elektrisch zu isolierende elektronische Bauteile auf, so können diese jeweils außenumfänglich von einer Glashülse umgeben sein oder es können mehrere elektronische Bauteile außenumfänglich von einer Glashülse umgeben sein, also in einer Glashülse mehrere elektronische Bauteile angeordnet sein.

Die das zumindest eine elektronische Bauteil außenumfänglich umgebende Hülse ist in dem Schaft zweckmäßigerweise derart angeordnet, dass ihre Mittelachse im Wesentlichen parallel zur Schaftmittelachse ausgerichtet ist, d. h. die Wandung der Glashülse ist zwischen dem elektronischen Bauteil mit seinem außenseitig liegenden elektrischen Leiter und der Außenwandung des Schaftes angeordnet und bildet somit einen zwischen dem elektronischen Bauteil und der Außenwandung des Schaftes angeordneten elektrischen Isolator, der einen Stromübergang von dem elektronischen Bauteil bzw. von dem außenseitig des Bauteils liegenden elektrischen Leiter auf die Außenwandung des Schaftes verhindert. Hierbei erweist es sich als vorteilhaft, dass die Glashülse aufgrund der guten elektrischen Isolationseigenschaften von Glas sehr dünnwandig ausgebildet sein kann und auch sehr dünnwandig herstellbar ist. Es kann somit eine elektrische Isolation des elektronischen Bauteils geschaffen werden, die sich in deutlich geringerem Maße auf die radialen Abmessungen des Schaftes auswirkt, als dies bei den aus dem Stand der Technik bekannten endoskopischen Schaftinstrumenten der in Rede stehenden Art der Fall ist.

Die Glashülse ist an ihrem distalen Ende von einer Abdeckung verschlossen. Diese gestalterische Maßnahme dient vorrangig zum Schutz eines in der Glashülse angeordneten elektronischen Bauteils und ist beispielsweise dann von Vorteil, wenn das distale Ende des erfindungsgemäßen Schaftinstrumentes in Richtung des elektronischen Bauteiles offen ausgebildet ist. In diesem Fall wird das Bauteil von der Abdeckung einerseits mechanisch geschützt und andererseits von gegebenenfalls in der Außenumgebung des distalen Schaftendes befindlichen Flüssigkeiten wie beispielsweise einer in Verbindung mit dem Schaftinstrument eingesetzten Spülflüssigkeit abgeschirmt, welche ansonsten auch einen unerwünschten Strompfad von dem elektronischen Bauteil zu einem mit dem Schaftinstrument zu behandelnden Patienten und unerwünschte Strompfade an dem elektronischen Bauteil selbst bilden könnte. Die Abdeckung ist daher vorzugsweise ebenfalls aus einem elektrisch nicht leitenden Material ausgebildet. Ein weiterer Vorteil der an dem distalen Ende der Glashülse angeordneten Abdeckung besteht darin, dass sie die möglichst dünnwandig ausgebildete Glashülse versteift und somit deren Stabilität erhöht.

Weiter bevorzugt ist die das distale Ende der Glashülse verschließende Abdeckung eine Glasabdeckung. Die Verwendung einer Glasabdeckung ist typischerweise dann zweckmäßig, wenn das in der Glashülse angeordnete elektronische Bauteil ein zur Emission oder Aufnahme von Licht bereitgestelltes Bauteil ist, wobei die Glasabdeckung einen Lichtdurchgang von dem elektronischen Bauteil zu der distalen Außenumgebung des Schaftinstrumentes und umgekehrt ermöglicht. Die Glashülse kann an ihren beiden Flachseiten planparallel eben ausgebildet sein oder zur Bildung eines lichtablenkenden optischen Bauteils, zueinander schräg ausgerichtete ebene Flachseiten aufweisen oder an mindestens einer ihrer Flachseiten eine konvexe oder konkave Wölbung aufweisen.

Gemäß der vorliegenden Erfindung sind die Glashülse und die Abdeckung separate Teile, welche stoffschlüssig miteinander verbunden sind. Zur stoffschlüssigen Verbindung von Glashülse und Abdeckung ist die Abdeckung mit der Glashülse verklebt. Dabei ist vorgesehen, dass die Abdeckung mit einem mit dem Innenquerschnitt der Glashülse korrespondierenden Abschnitt in die Glashülse eingreift und in dieser Position mit der Glashülse verklebt ist, wobei der in das distale Ende der Hülse eingreifende Absatz die Stabilität der Glashülse in besonderem Maße erhöht. Die Verwendung von separaten, die Glashülse und die Abdeckung bildenden Teilen ist beispielsweise dann angebracht, wenn eine Abdeckung vorgesehen ist, die aus einem anderen Material als Glas oder aus einer Kombination mehrerer Materialen ausgebildet ist. Unabhängig davon, ob die Abdeckung aus Glas oder einem anderen Material bzw. anderen Materialien ausgebildet ist, erweist es sich aber als vorteilhaft, dass eine distalseitig von einer Abdeckung verschlossene Glashülse bei Verwendung zweier separater Teile verhältnismäßig einfach herstellbar ist, indem lediglich auf einem die Glashülse bildenden Glasröhrchen ein die Abdeckung bildendes Plättchen aufgeklebt werden muss. Hinsichtlich einer sicheren, spannungsfreien Klebverbindung zwischen der Glashülse und der Abdeckung ist es von Vorteil, wenn die Abdeckung aus Glas ausgebildet ist und somit mit der Glashülse identische oder zumindest weitestgehend identische Werkstoffeigenschaften wie beispielsweise einen zumindest annähernd gleichen Ausdehnungskoeffizienten aufweist.

Vorteilhaft kann die Glashülse einen kreisförmigen Querschnitt aufweisen. D. h., die Glashülse wird vorzugsweise von einem hohlzylindrischen Glasrohr gebildet, welches kostengünstig herstellbar und im Handel als Halbzeug kostengünstig erhältlich ist.

Neben der Verwendung einer Glashülse mit einem kreisförmigen Querschnitt sieht die Erfindung vorteilhaft auch Ausgestaltungen der Glashülse vor, bei welchen diese einen von einer Kreisform abweichenden Querschnitt aufweist. Hierbei kann die Querschnittsform der Glashülse, die generell beliebig ist, z. B. mit den Querschnittsabmessungen des darin angeordneten elektronischen Bauteils korrespondieren. Ferner kann die Querschnittsform der Glashülse auch an weitere Komponenten des endoskopischen Instrumentes, welche in Querschnittsrichtung des Schaftes neben der Glashülse angeordnet sind bzw. an durch den Schaft verlaufende Kanäle, wie beispielsweise Arbeitskanäle zur Durchführung von Hilfsinstrumenten und/oder Spülflüssigkeit, angepasst werden. Insbesondere in diesem Zusammenhang ist bevorzugt vorgesehen, dass die Glashülse einen sichelförmigen Querschnitt aufweist. Hierbei kann die Glashülse an ihrer konvex gewölbten Außenseite einen Außenradius aufweisen, welcher mit dem Innenradius des Schaftes des Schaftinstrumentes korrespondiert, sodass die Glashülse in dem Schaft unmittelbar an der Innenwandung des Schaftes anliegend angeordnet werden kann, wobei ein der konvex gewölbten Außenseite gegenüberliegender konkav gewölbter Bereich der Außenseite dazu führt, dass in dem Schaft außenseitig des konkav gewölbten Bereichs ein vergleichsweise großer Raum für die Anordnung weiterer Komponenten des Schaftinstruments zur Verfügung steht.

Bevorzugt ist das zumindest eine außenumfänglich von einer Glashülse umgebende Bauteil eine LED. Diese LED dient vorteilhaft zur Ausleuchtung eines distalseitig des Schaftinstrumentes befindlichen Bereiches. Die vorzugsweise vorgesehene Abdeckung, welche die Glashülse an ihrem distalen Ende verschließt, ist hierbei zweckmäßigerweise aus einem lichttransparenten Material und bevorzugt aus Glas ausgebildet. Bei der LED handelt es sich weiter bevorzugt um einen LED-Chip, welcher zumindest einen außenseitig der LED angeordneten Leiter aufweist, der einen an der lichtemittierenden Seite an der LED vorgesehenen Anschlusskontakt mit einem Pol einer Gleichspannungsquelle leitungsverbindet. Die Glashülse und deren distalseitige Abdeckung schirmen die LED und insbesondere deren außenseitig liegenden Leiter gegenüber dem Schaft des Schaftinstrumentes elektrisch isolierend ab, wobei die Glashülse und deren Abdeckung auch in gewissem Grade thermisch isolierend wirken, so dass die von der LED abgestrahlte Wärme vor allem bei gleichzeitiger Wärmeabfuhr durch das Innere des Schaftes in proximaler Richtung nicht oder nur in geringem Maße in die radiale und distale Außenumgebung der Glashülse gelangt.

Gemäß einer weiteren bevorzugten Weiterbildung der Erfindung sind an der vorzugsweise die Glashülse an ihrem distalen Ende verschließenden Abdeckung mehrere optische Linsen ausgebildet. Dementsprechend sind an der Abdeckung über deren Querschnittsfläche verteilt eine Vielzahl von Linsen vorgesehen, die bei einer Abdeckung aus einem lichttransparenten Material durch einen entsprechenden Anschliff der Abdeckung ausgebildet sein können, gegebenenfalls aber auch von separaten Teilen gebildet werden können, welche in der Abdeckung eingesetzt sind oder auf die Abdeckung aufgesetzt sind.

Bei den an der Abdeckung ausgebildeten Linsen kann es sich sowohl um Sammel- als auch um Zerstreuungslinsen handeln. Die Verwendung von Zerstreuungslinsen ist insbesondere in Verbindung mit einer in der Glashülse angeordneten LED einer Beleuchtungseinrichtung vorteilhaft, bei welcher mit der Zerstreuungslinse eine homogene Verteilung des von der LED emittierten Lichtes bewirkt werden kann. So sind die Linsen bevorzugt konkave Linsen und vorzugsweise plan-konkave Linsen. In letztgenanntem Fall ist vorrangig vorgesehen, dass an der aus einem lichtdurchlässigen Material ausgebildeten Abdeckung an der der Glashülse zugewandten Flachseite mehrere konkave Vertiefungen ausgebildet sind.

Nachfolgend ist die Erfindung anhand von in den Zeichnungen dargestellten Ausführungsbeispielen näher erläutert. In den Zeichnungen zeigt jeweils schematisch stark vereinfacht und in unterschiedlichen Maßstäben:
- Fig. 1:: in einer Draufsicht ein distales Ende eines endoskopischen Schaftinstrumentes,
- Fig. 2:: in einer geschnittenen Seitenansicht eine in einer Glashülse angeordnete LED des Schaftinstrumentes nach Fig. 1,
- Fig. 3:: in perspektivischer Darstellung eine Glashülse zur Aufnahme einer LED gemäß einer zweiten Ausgestaltung,
- Fig. 4:: die Glashülse nach Fig. 3 in einer geschnittenen Seitenansicht und
- Fig. 5:: die Glashülse nach Fig. 3 in einer Untersicht.

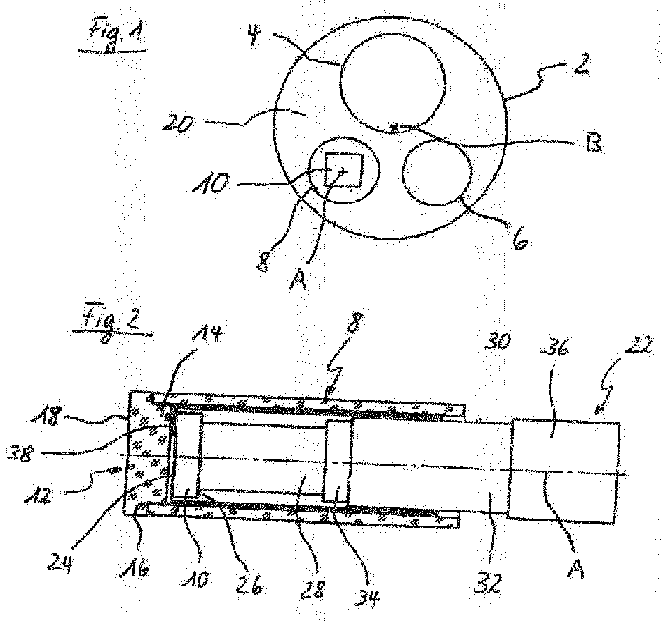

Fig. 1 zeigt das distale Ende eines Schaftes 2 eines endoskopischen Schaftinstruments. An diesem distalen Ende des Schaftes 2 mündet ein durch den gesamten Schaft 2 verlaufender Arbeitskanal 4. Der Arbeitskanal 4 dient vorrangig dazu, ein in Verbindung mit dem Schaftinstrument eingesetztes, nicht dargestelltes Hilfsinstrument zu einem distalseitig des Schaftes 2 gelegenen Einsatzort zu führen. Seitlich neben dem Arbeitskanal 4 sind im Bereich des distalen Endes des Schaftes 2 eine Bildsensoreinheit 6 und in einer Glashülse 8 ein elektronisches Bauteil 10 in Form eines LED-Chips angeordnet. Der LED-Chip dient zur Ausleuchtung eines distalseitig des Schaftes 2 gelegenen Bereichs.

Die in Fig. 2 detailliert dargestellte Glashülse 8, welche den darin angeordneten LED-Chip gegenüber dessen Außenumgebung elektrisch isoliert, ist hohlzylindrisch ausgebildet und an ihrem distalen Ende von einer Abdeckung 12 verschlossen. Mit einem an der Abdeckung 12 axial vorstehenden zylindrischen Vorsprung 14, dessen Außendurchmesser mit dem Innendurchmesser der Glashülse 8 korrespondiert, greift die aus Glas ausgebildete Abdeckung 12 in das distale Ende der Glashülse 8 ein, wobei ein gegenüber dem Vorsprung 14 radial erweiterter Abschnitt 16 auf dem distalen Ende der Glashülse 8 aufliegt. In dieser Position ist die Abdeckung 12 mit der Glashülse 8 mittels einer Klebverbindung stoffschlüssig verbunden.

In dem Schaft 2 ist die Glashülse 8 derart angeordnet, dass eine Mittelachse A der Glashülse 8 parallel zu einer Mittelachse B des Schaftes 2 ausgerichtet ist. Zudem ist die Glashülse 8 in dem Schaft 2 so angeordnet, dass eine Außenseite 18 der die Glashülse 8 distalseitig verschließenden Abdeckung 12 bündig mit einer distalen Stirnseite 20 des Schaftes 2 fluchtet.

Der LED-Chip ist in der Glashülse 8 mit geringem Abstand zu der Abdeckung 12 angeordnet und stützt sich proximalseitig auf einem Koaxialkabel 22 ab, welches durch den gesamten Schaft 2 geführt ist und den LED-Chip mit einer proximalseitig des Schaftes 2 angeordneten, in der Zeichnung nicht dargestellten Gleichspannungsquelle verbindet. In Fig. 2 ist das Koaxialkabel 22 geschnitten dargestellt, so dass ein Innenleiter 28, eine den Innenleiter 22 außenseitig umgebende Isolationsummantelung 34, ein Außenleiter 32 sowie eine äußere Isolationsummantelung 36 des Koaxialkabels 22 deutlich werden. Zur elektrischen Verbindung des LED-Chips mit der Gleichspannungsquelle weist der LED-Chip an seiner lichtemittierenden distalen Seite 24 und an seiner proximalen Seite 26 jeweils einen Anschlusskontakt auf, wobei die Anschlusskontakte ebenfalls aus der Zeichnung nicht ersichtlich sind. Der an der proximalen Seite 26 des LED-Chips ausgebildete Anschlusskontakt wird von dem Innenleiter 28 des Koaxialkabels 22 kontaktiert, während der an der distalen Seite 24 des LED-Chips angeordnete Anschlusskontakt über einen Leiter 30 mit einem Außenleiter 32 des Koaxialkabels 22 elektrisch leitend verbunden ist.

Der den LED-Chip mit dem Außenleiter 32 des Koaxialkabels 22 verbindende Leiter 30 wird von einer metallischen Hülse gebildet. Diese Hülse ist derart angeordnet, dass sie den LED-Chip außenseitig, d. h. außenumfänglich umgibt und auch den Außenleiter 32 des Koaxialkabels 22 kontaktierend in einem distalen Endabschnitt des Koaxialkabels 22 außenseitig umgibt. Hierbei wird die Hülse radial außenseitig vollständig von der Glashülse 8 überdeckt. An ihrem distalen Ende weist die den Leiter 30 bildende Hülse einen gegenüber der übrigen Hülse vorstehenden Steg 38 auf, welcher zur Kontaktierung des an der distalen Seite 24 des LED-Chips angeordneten Anschlusskontakts quer zur Längsausdehnung der Hülse abgewinkelt ist.

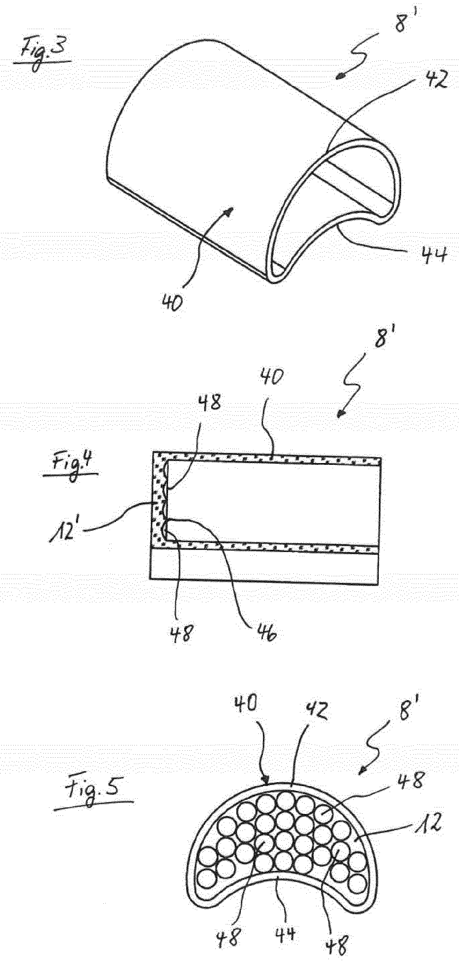

Die in den Fig. 3 - 5 dargestellte Glashülse 8' dient ebenfalls zur elektrischen Abschirmung zumindest eines in der Glashülse 8' angeordneten LED-Chips 10 gegenüber dessen Außenumgebung. Die Glashülse 8' ist monolithisch ausgebildet, wobei ein Umfangsteil 40 an seinem distalen Ende von einer Abdeckung 12' verschlossen ist. Der Querschnitt der Glashülse 8' ist sichelförmig ausgebildet, wobei ein konvex gewölbter Wandungsbereich 42 abgerundet in einen konkav gewölbten Wandungsbereich 44 übergeht. An einer proximalen Seite 46 der Abdeckung 12' ist eine Vielzahl von konkav gewölbten Vertiefungen 48 ausgebildet, welche jeweils eine plan-konkave Linse bilden. Diese plankonkaven Linsen dienen dazu, das von zumindest einem in der Glashülse 8' angeordneten LED-Chip emittierte Licht distalseitig der Glashülse 8' zu streuen, um so eine homogene Lichtverteilung zu bewirken.

### Bezugszeichen

- 2: Schaft
- 4: Arbeitskanal
- 6: Bildsensoreinheit
- 8,8': Glashülse
- 10: elektronisches Bauteil
- 12, 12': Abdeckung
- 14: Vorsprung
- 16: Abschnitt
- 18: Außenseite
- 20: Stirnseite
- 22: Koaxialkabel
- 24: Seite
- 26: Seite
- 28: Innenleiter
- 30: Leiter
- 32: Außenleiter
- 34: Isolationsummantelung
- 36: Isolationsummantelung
- 38: Steg
- 40: Umfangsteil
- 42: Wandungsbereich
- 44: Wandungsbereich
- 46: Seite
- 48: Vertiefung

- A: Mittelachse
- B: Mittelachse

## Patentansprüche

1. Endoskopisches Schaftinstrument, welches im Bereich seines distalen Endes ein elektronisches Bauteil (10) mit einem außenseitig liegenden elektrischen Leiter (30) aufweist, wobei das elektronische Bauteil (10) zumindest außenumfänglich von einer Glashülse (8, 8') umgeben ist, wobei die Glashülse (8,8') an ihrem distalen Ende von einer lichttransparenten Abdeckung (12, 12') verschlossen ist, **dadurch gekennzeichnet, dass** die Abdeckung (12, 12') mit einem mit dem Innenquerschnitt der Glashülse (8, 8') korrespondierenden Abschnitt in die Glashülse eingreift und in dieser Position mit der Glashülse verklebt ist, wobei die Glashülse (8, 8') und die Abdeckung (12, 12') das darin angeordnete elektronische Bauteil (10) gegenüber dessen Außenumgebung elektrisch isolieren.

2. Endoskopisches Schaftinstrument nach Anspruch 1, **dadurch gekennzeichnet, dass** die Abdeckung (12, 12') eine Glasabdeckung ist.

3. Endoskopisches Schaftinstrument nach einem der vorangehenden Ansprüche, **dadurch gekennzeichnet, dass** die Glashülse (8) einen kreisförmigen Querschnitt aufweist.

4. Endoskopisches Schaftinstrument nach einem der Ansprüche 1 oder 2, **dadurch gekennzeichnet, dass** die Glashülse (8') einen von einer Kreisform abweichenden und vorzugsweise sichelförmigen Querschnitt aufweist.

5. Endoskopisches Schaftinstrument nach einem der vorangehenden Ansprüche, **dadurch gekennzeichnet, dass** das elektronische Bauteil (10) eine LED ist.

6. Endoskopisches Schaftinstrument nach einem der vorangehenden Ansprüche, **dadurch gekennzeichnet, dass** an der Abdeckung (12, 12') mehrere optische Linsen ausgebildet sind.

7. Endoskopisches Schaftinstrument nach Anspruch 6, **dadurch gekennzeichnet, dass** die Linsen plan-konkave Linsen sind.

## Claims

1. An endoscopic shank instrument which in the region of its distal end comprises an electronic component (10) with an electrical lead (30) which lies at the outer side, wherein the electronic component (10) at least at the outer periphery is surrounded by a glass sleeve (8, 8'), wherein the glass sleeve (8, 8') at its distal end is closed by a light-transparent covering (12, 12'), **characterised in that** the covering (12, 12'), with a section which corresponds to the inner cross section of the glass sleeve (8, 8'), engages into the glass sleeve and is bonded to the glass sleeve in this position, wherein the glass sleeve (8, 8') and the covering (12, 12') electrically insulate the electronic component (10) which is arranged therein, with respect to its outer surroundings.

2. An endoscopic shank instrument according to claim 1, **characterised in that** the covering (12, 12') is a glass covering.

3. An endoscopic shank instrument according to one of the preceding claims, **characterised in that** the glass sleeve (8) has a circular cross section.

4. An endoscopic shank instrument according to one of the claims 1 or 2, **characterised in that** the glass sleeve (8') has a cross section which differs from a circular shape and is preferably crescent-shaped.

5. An endoscopic shank instrument according to one of the preceding claims, **characterised in that** the electronic component (10) is an LED.

6. An endoscopic shank instrument according to one of the preceding claims, **characterised in that** several optical lenses are formed on the covering (12, 12').

7. An endoscopic shank instrument according to claim 6, **characterised in that** the lenses are planar-concave.

## Revendications

1. Instrument à tige endoscopique, qui présente, dans la région de son extrémité distale, un composant électronique (10) pourvu d'un conducteur électrique (30) situé côté extérieur, le composant électronique (10) étant entouré, au moins sur sa circonférence extérieure, d'un manchon en verre (8, 8'), le manchon en verre (8, 8') étant fermé à son extrémité distale par un couvercle (12, 12') transparent à la lumière, **caractérisé en ce que** le couvercle (12, 12') s'engage dans le manchon en verre par une partie correspondant à la section transversale intérieure du manchon en verre (8, 8') et est collé dans cette position au manchon en verre, le manchon en verre (8, 8') et le couvercle (12, 12') isolant électriquement de son environnement extérieur le composant électronique (10) disposé à l'intérieur.

2. Instrument à tige endoscopique selon la revendication 1, **caractérisé en ce que** le couvercle (12, 12') est un couvercle en verre.

3. Instrument à tige endoscopique selon l'une des revendications précédentes, **caractérisé en ce que** le manchon en verre (8) présente une section transversale circulaire.

4. Instrument à tige endoscopique selon l'une des revendications 1 ou 2, **caractérisé en ce que** le manchon en verre (8') présente une section transversale différente d'une forme circulaire et de préférence en forme de croissant.

5. Instrument à tige endoscopique selon l'une des revendications précédentes, **caractérisé en ce que** le composant électronique (10) est une diode électroluminescente.

6. Instrument à tige endoscopique selon l'une des revendications précédentes, **caractérisé en ce que** sont formées sur le couvercle (12, 12') plusieurs lentilles optiques.

7. Instrument à tige endoscopique selon la revendication 6, **caractérisé en ce que** les lentilles sont des lentilles plan-concaves.
